# EUROPEAN PATENT APPLICATION

(11) **EP 4 775 143 A1**
(43) Date of publication of application: **15.07.2026**
(21) Application number: 25220186.8
(22) Date of filing: 02.12.2025
(51) Int. Cl.: A61B 5/103, A43B 3/44, A61B 5/11, A61B 5/00

(54) **SMART SOLE ARCHITECTURE FOR MEASURING GROUND REACTION FORCE AND METHOD FOR UNIFYING COORDINATES FOR FEET POSITIONS AND GESTURES**

(30) Priority: 10.01.2025 US 202519017350
(71) Applicant: Hitachi, Ltd., Tokyo 100-8280 (JP)
(72) Inventor: ZHOU, Quan, Tokyo, 100-8280 (JP); ABDOLLAHI, Masoud, Tokyo, 100-8280 (JP)
(74) Representative: Mewburn Ellis LLP

(57) **Abstract**

A pressure profiling method comprising receiving, by a processor, raw data from a plurality of sensors embedded in a pair of shoes being used by a user; performing, by the processor, preprocessing of the raw data to generate preprocessed data; calculating, by the processor, ground reaction force on the user using the preprocessed data; and performing, by the processor, at least one of posture adjustment recommendation generation, report generation, or additional data analysis using the ground reaction force derived from the preprocessed data.

## Description

### BACKGROUND

### Field

The present disclosure is generally directed to methods and systems for performing pressure profiling and coordinate unification.

### Related Art

Measuring foot pressure is important for a number of reasons (e.g., wound prevention, gait monitoring and treatment, etc.) Conventional smart insoles/shoes are capable of measuring the plantar pressure or vertical force underneath user's feet. However, in order to understand the musculoskeletal system of a user, measurement of the user's ground reaction force (GRF) is essential, which is not tracked/measured by conventional smart insoles/shoes.

In the related art, a method utilizing one or more sensors mounted above shoe insoles for measuring the shear component of a force is disclosed. While the method discloses horizontal force tracking, the overall shear force being applied to the body is not measured/computed. The overall shear force applying to the body is resultant of shear force underneath the foot and other shear forces applying by the shoe body to the other parts of the foot (e.g., sides and top of the foot), and goes beyond horizontal force that is measured above the insole level.

Currently, there is no technology available for measuring the shear (horizontal) force underneath the feet. Specifically, conventional technologies only focus on pressure distribution directly under the human foot. There is no clear quantitative measurement of the GRF between the feet and the ground. Utilizing smart insoles to quantify GRF could open doors to comprehensive musculoskeletal assessment of the human body in natural environments. Such data and analysis provide not only important insights into industrial safety, but ergonomics in operations as well.

There exists a need for a multilayer insole structure that can be used to measure the GRF between the feet and the ground. The measured GRF can used to monitor and analyze ergonomics in industrial operations, preventing potential injuries.

In addition, while conventional smart insoles/shoes are capable of measuring the plantar pressure or vertical force underneath feet and report them in the local coordinate system of each foot's insole, in order to provide a more comprehensive evaluation of the posture and musculoskeletal system, information such as relative position of the feet is critical in performing such evaluations. There is currently no technology available to measure the pressure and force underneath the feet and report them as a single unique coordinate system.

In the related art, a method for tracking the location/movement of each human foot with sensors like IMU (Inertial Measurement Unit) is disclosed. However, the method fails to establish the correlation between both human feet and is not able to further track and analyze the correlation.

There exists a need for a method that is capable of reporting the relative location of each insole within a unique coordinate system. Information derived from the unique coordinate system can then be used to calculate the precise position of the overall Center of Pressure (COP) that originates from both feet, which is critical in performing comprehensive biomechanical analysis of the whole body.

### SUMMARY

Aspects of the present disclosure involve an innovative method for performing pressure profiling. The method may include receiving, by a processor, raw data from a plurality of sensors embedded in a pair of shoes being used by a user; performing, by the processor, preprocessing of the raw data to generate preprocessed data; calculating, by the processor, ground reaction force on the user using the preprocessed data; and performing, by the processor, at least one of posture adjustment recommendation generation, report generation, or additional data analysis using the ground reaction force derived from the preprocessed data.

Aspects of the present disclosure involve an innovative non-transitory computer readable medium, storing instructions for performing pressure profiling. The instructions may include receiving raw data from a plurality of sensors embedded in a pair of shoes being used by a user; performing preprocessing of the raw data to generate preprocessed data; calculating ground reaction force on the user using the preprocessed data; and performing at least one of posture adjustment recommendation generation, report generation, or additional data analysis using the ground reaction force derived from the preprocessed data.

Aspects of the present disclosure involve an innovative server system for performing pressure profiling. The system may include a memory; and a processor in communication with the memory, wherein the processor is configured to receive raw data from a plurality of sensors embedded in a pair of shoes being used by a user; perform preprocessing of the raw data to generate preprocessed data; calculate ground reaction force on the user using the preprocessed data; and perform at least one of posture adjustment recommendation generation, report generation, or additional data analysis using the ground reaction force derived from the preprocessed data.

Aspects of the present disclosure involve an innovative system for performing pressure profiling. The system may include a memory; and a processor in communication with the memory, wherein the processor is configured to receive raw data from a plurality of sensors embedded in a pair of shoes being used by a user; perform preprocessing of the raw data to generate preprocessed data; calculate ground reaction force on the user using the preprocessed data; and perform at least one of posture adjustment recommendation generation, report generation, or additional data analysis using the ground reaction force derived from the preprocessed data.

Aspects of the present disclosure involve an innovative method for performing coordinate unification. The method may include receiving, by a processor, data from a plurality of sensors embedded in a pair of shoes while the pair of shoes are being used in one or more standardized activities by a user; determining, by the processor, relative position of feet of the user based on the data; generating, by the processor, a unified coordinate model from the relative position of the feet of the user; and calculating, by the processor, a position of an overall Center of Pressure (CoP) of the user based on the unified coordinate model.

In some example implementations, the method may further include determining, by the processor, orientation of the feet of the user based on the data, wherein the processor is configured to generate the unified coordinate model from both the relative position of the feet of the user and the orientation of the feet.

In some example implementations, the method may further include performing, by the processor, at least one of posture adjustment recommendation generation, evaluation report generation, or rehabilitation recommendation generation using the position of the overall CoP of the user.

Aspects of the present disclosure involve an innovative non-transitory computer readable medium, storing instructions for performing coordinate unification. The instructions may include receiving data from a plurality of sensors embedded in a pair of shoes while the pair of shoes are being used in one or more standardized activities by a user; determining relative position of feet of the user based on the data; generating a unified coordinate model from the relative position of the feet of the user; and calculating a position of an overall Center of Pressure (CoP) of the user based on the unified coordinate model.

In some example implementations, the instructions may further include determining orientation of the feet of the user based on the data, wherein the processor is configured to generate the unified coordinate model from both the relative position of the feet of the user and the orientation of the feet.

In some example implementations, the instructions may further include performing at least one of posture adjustment recommendation generation, evaluation report generation, or rehabilitation recommendation generation using the position of the overall CoP of the user.

Aspects of the present disclosure involve an innovative server system for performing coordinate unification. The system may include a memory; and a processor in communication with the memory, wherein the processor is configured to receive data from a plurality of sensors embedded in a pair of shoes while the pair of shoes are being used in one or more standardized activities by a user; determine relative position of feet of the user based on the data; generate a unified coordinate model from the relative position of the feet of the user; and calculate a position of an overall Center of Pressure (CoP) of the user based on the unified coordinate model.

In some example implementations, the processor is further configured to determine orientation of the feet of the user based on the data, wherein the processor is configured to generate the unified coordinate model from both the relative position of the feet of the user and the orientation of the feet.

In some example implementations, the processor is further configured to perform at least one of posture adjustment recommendation generation, evaluation report generation, or rehabilitation recommendation generation using the position of the overall CoP of the user.

Aspects of the present disclosure involve an innovative system for performing coordinate unification. The system may include a memory; and a processor in communication with the memory, wherein the processor is configured to receive data from a plurality of sensors embedded in a pair of shoes while the pair of shoes are being used in one or more standardized activities by a user; determine relative position of feet of the user based on the data; generate a unified coordinate model from the relative position of the feet of the user; and calculate a position of an overall Center of Pressure (CoP) of the user based on the unified coordinate model.

In some example implementations, the processor is further configured to determine orientation of the feet of the user based on the data, wherein the processor is configured to generate the unified coordinate model from both the relative position of the feet of the user and the orientation of the feet.

In some example implementations, the processor is further configured to perform at least one of posture adjustment recommendation generation, evaluation report generation, or rehabilitation recommendation generation using the position of the overall CoP of the user.

### BRIEF DESCRIPTION OF DRAWINGS

A general architecture that implements the various features of the disclosure will now be described with reference to the drawings. The drawings and the associated descriptions are provided to illustrate example implementations of the disclosure and not to limit the scope of the disclosure. Throughout the drawings, reference numbers are reused to indicate correspondence between referenced elements.
FIG. 1 illustrates an example schematic of a shoe utilizing a multi-layer smart sole system, in accordance with an example implementation.
FIG. 2 illustrates an example system architecture 200 of the smart sole system 104, in accordance with an example implementation.
FIG. 3 illustrates an example process flow 300 for performing pressure profiling, in accordance with an example implementation.
FIG. 4 illustrates an example display 400, in accordance with an example implementation.
FIG. 5 illustrates two conventional smart sole systems 500.
FIG. 6 illustrates an example system architecture 600 of a Unified Coordinate System (UCS) , in accordance with an example implementation.
FIG. 7 illustrates an example smart sole system 700 utilizing the sensors 602, in accordance with an example implementation.
FIG. 8 illustrates an example process flow 800 for performing coordinate unification, in accordance with an example implementation.
FIG. 9 illustrates an example display 900, in accordance with an example implementation.
FIG. 10 illustrates an example computing environment with an example computer device suitable for use in some example implementations.

### DETAILED DESCRIPTION

The following detailed description provides details of the figures and example implementations of the present application. Reference numerals and descriptions of redundant elements between figures are omitted for clarity. Terms used throughout the description are provided as examples and are not intended to be limiting. For example, the use of the term "automatic" may involve fully automatic or semi-automatic implementations involving user or administrator control over certain aspects of the implementation, depending on the desired implementation of one of the ordinary skills in the art practicing implementations of the present application. Selection can be conducted by a user through a user interface or other input means, or can be implemented through a desired algorithm. Example implementations as described herein can be utilized either singularly or in combination and the functionality of the example implementations can be implemented through any means according to the desired implementations.

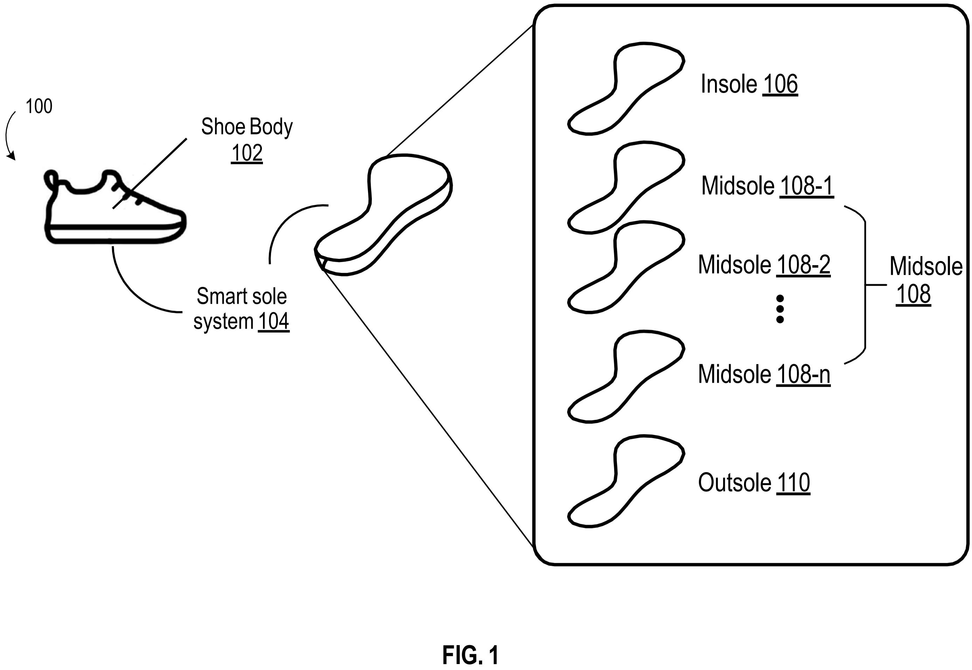

FIG. 1 illustrates an example schematic of a shoe utilizing a multi-layer smart sole system, in accordance with an example implementation. As illustrated in FIG. 1, a shoe 100 may include components such as, but not limited to, a shoe body 102, a smart sole system 104, etc. The smart sole system 104 may form an integral part of the shoe 100 or be one or more separate units/modules that can be used through insertion into common/conventional shoes.

The smart sole system 104 may include components such as, but not limited to, an insole 106, a midsole 108, an outsole 110, etc. A number of sensors are embedded in the insole 106 and one or more of the midsole 108 or outsole 110. The sensors are used to captures a pressure profile between feet of the user and an external contacting surface of shoes utilizing the smart sole system 104 as raw data. The raw data may include data such as, but not limited to, data measuring vertical reaction force on feet of the user, data measuring both the vertical reaction force and shear reaction force on the feet of the user, etc. The embedded sensors may include either (i) pressure sensors; or (ii) a combination of pressure sensors and one or more of inertial measuring unit (IMU) sensors, temperature sensors, or humidity sensors. The pressure sensors are used to measure the vertical reaction force (e.g. pressure) exerted on the feet. In some example implementations, three-dimensional reaction forces can be generated/measured through inclusion of additional sensors to the pressure sensors such that both vertical and shear reaction forces can be tracked.

The midsole 108 of a smart sole system 104 may include one or more midsole layers 108-1, 108-2, ... 108-n. Each layer of the midsole layers 108-1 to 108-n may be imbedded with any combination of one or more of sensors such as, but not limited to, a pressure sensor, an IMU sensor, a temperature sensor, a humidity sensor, etc.

When utilized in a pair of shoes, the smart sole system 104 captures a three-dimensional pressure profile between the feet of the user and the external contacting surface of the shoes, from which the ground reaction force (GRF) on the whole human body can be derived. Specifically, a mathematical distribution function in the vertical direction (normal to the ground) is applied to the pressure mapping information received from (i) the insole 106 and (ii) the midsole 108 and/or the outsole 110, to reconstruct a three-dimensional (3D) pressure profile(s) throughout of the entire sole volume (between the ground and the bottom of the foot). The vertical components of the GRF can be derived/calculated by integrating the pressure mapping information/data over the sole area. The shear components of the GRF can be derived/calculated from the 3D pressure profile(s) of the sole(s). The overall GRF is the resultant vector of the vertical components and the shear components, which is the force at the sole/ground interface.

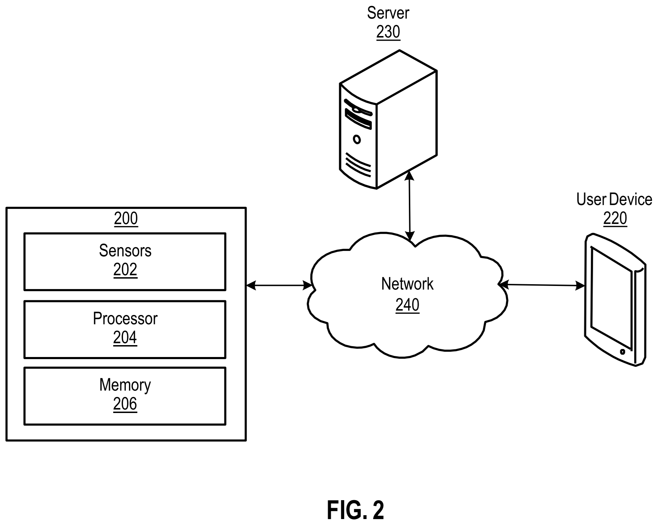

FIG. 2 illustrates an example system architecture 200 of the smart sole system 104, in accordance with an example implementation. As illustrated in FIG. 2, the smart sole system 104 may include components such as, but not limited to, sensors 202, a processor 204, a memory 206, etc. In some alternate implementations, different and/or additional components may be included in the smart sole system 104.

The sensors 202 may be embedded in the insole 106 and one or more of the midsole 108 or outsole 110 of FIG. 1. The sensors 202 may include either (i) pressure sensors; or (ii) a combination of pressure sensors and one or more of inertial measuring unit (IMU) sensors, temperature sensors, humidity sensors, etc. The pressure sensors are used to measure the vertical reaction force (e.g. pressure) exerted on the feet. Three-dimensional reaction forces can be generated/measured through inclusion of additional sensors to the pressure sensors such that both vertical and shear reaction forces can be tracked. The measured sensor data is then transmitted from the sensors 202 to the processor 204.

The processor 204 performs data processing on the data collected from the sensors 202 and calculates the GRF on the human body. In some example implementations, the processor 204 performs preprocessing on the data received from the sensors 202, which may include one or more processes such as, but not limited, analog/digital data conversion, data reduction, data or feature extraction, data transformation, data cleaning, normalization, etc. In some example implementations, the processor 204 may perform additional data analysis such as fatigue monitoring, load assessment, and performance tracking, using the data collected from the sensors 202.

The memory 206 stores instructions to be performed by the processor 204, the collected data from the sensors 202, the data calculated by the processor 204 (e.g., GRF, etc.), instructions/programs that are being used in the various components of the smart sole system 104, etc. The memory 206 may be any storage medium such as, but not limited to, read-only memories, random access memories, solid-state devices and drives, or any other types of tangible or non-transitory media suitable for storing electronic information.

A user device 220 communicates with the system architecture 200 through a network 240 to receive information from the system architecture 200. Network 240 can be any network or combination of networks (e.g., internet, local area network, wide area network, telephonic network, cellular network, satellite network, etc.). In some example implementations, request for performing GRF computation/calculation may be issued by a user/operator through a user device 220 and received at the processor 204.

Examples of the user device 220 may include, but are not limited to, highly mobile devices (e.g., smartphones, devices in vehicles and other machines, devices carried by humans and animals, and the like), mobile devices (e.g., tablets, notebooks, laptops, personal computers, portable televisions, radios, and the like), and devices not designed for mobility (e.g., desktop computers, other computers, information kiosks, televisions with one or more processors embedded therein and/or coupled thereto, radios, and the like).

In some example implementations, an Artificial Intelligence (AI) model may be stored in the memory 206, and used by the processor 204 to generate recommendations based on the computed GRF in real-time. Recommendation may include, but not limited to, one or more of position adjustment or movement adjustment to reduce impact forces, product recommendation to minimize GRF, exercises to improve body alignment, etc.

In some example implementations, the derivation and training of the AI model may be performed through a server 230 through use of historical data (e.g., sensor data, previously generated recommendations, etc.). The trained AI model may then be transmitted from the server 230 and received by the smart sole system 104 to be stored in the memory 206. Communication between the server 230 and the system architecture 200 is facilitated through the network 240. The AI model may include, but not limited to, a convolutional neural network (CNN), a recurrent neural network (RNN), a deep RNN (DRNN), a Q-learning network (QN), a deep Q-learning network (DQN), decision trees, a K-Nearest Neighbors, etc. RNN may include long short-term memory (LSTM), etc.

In alternate implementations, instead of storing the AI model in the memory 206, the calculated GRF and associated data are transmitted to the server 230 for recommendation generation. Specifically, recommendation generation is performed through the AI model stored on the server 230. In alternate implementations, a remote storage may be used instead of the memory 206. For example, cloud storage may be utilized in performing the data storage function.

In some example implementations, the calculated GRF, associated information/data, and/or recommendations are transmitted to the user device 220 for review. The information and recommendations may be presented in real-time through a graphic user interface (GUI) on the user device 220 as graphs, charts, texts, etc.

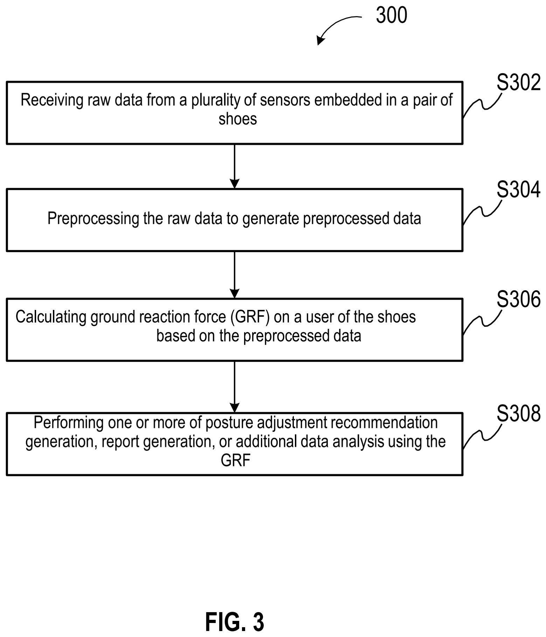

FIG. 3 illustrates an example process flow 300 for performing pressure profiling, in accordance with an example implementation. The process flow 300 begins at step S302 where raw data is received from a plurality of sensors embedded in a pair of shoes being used by a user. The raw data may include data such as, but not limited to, data measuring vertical reaction force on feet of the user, data measuring both the vertical reaction force and shear reaction force on the feet of the user, etc. At step S304, the raw data is then preprocessed to generate preprocessed data. The raw data may be preprocessed by performing one or more of data cleaning, data organization, data transformation, data normalization, data reduction, etc.

The process then continues to step S306 where ground reaction force on the user is calculated using the preprocessed data. At step S308, at least one of posture adjustment recommendation generation, report generation, or additional data analysis is performed using the ground reaction force derived from the preprocessed data. In some example implementations, an Artificial Intelligence (AI) model may be derived and utilized in performing one or more of posture adjustment recommendation generation, report generation, or additional/advanced data analysis.

The AI model may include, but not limited to, recurrent neural network (RNN), deep RNN (DRNN), Q-learning network (QN), deep Q-learning network (DQN), decision trees, K-Nearest Neighbors, etc. RNN may include long short-term memory (LSTM), etc. In some example implementations, the AI model is a large multimodal language model that works with different types of input data, such as text, images, audio, video, etc. The AI model is iteratively trained using historical data as training input and training parameters are adjusted to generate the posture adjustment recommendation, analysis report, and additional/advanced data analysis.

In some example implementations, the posture adjustment recommendation, analysis report, and additional/advanced data analysis may be transmitted to a user device (e.g., user device 220) to be reviewed by the user. Examples of the user device may include, but are not limited to, mobile devices (e.g., smartphones, tablets, notebooks, laptops, personal computers, etc.) and devices not designed for mobility (e.g., desktop computers, information kiosks, televisions, etc.).

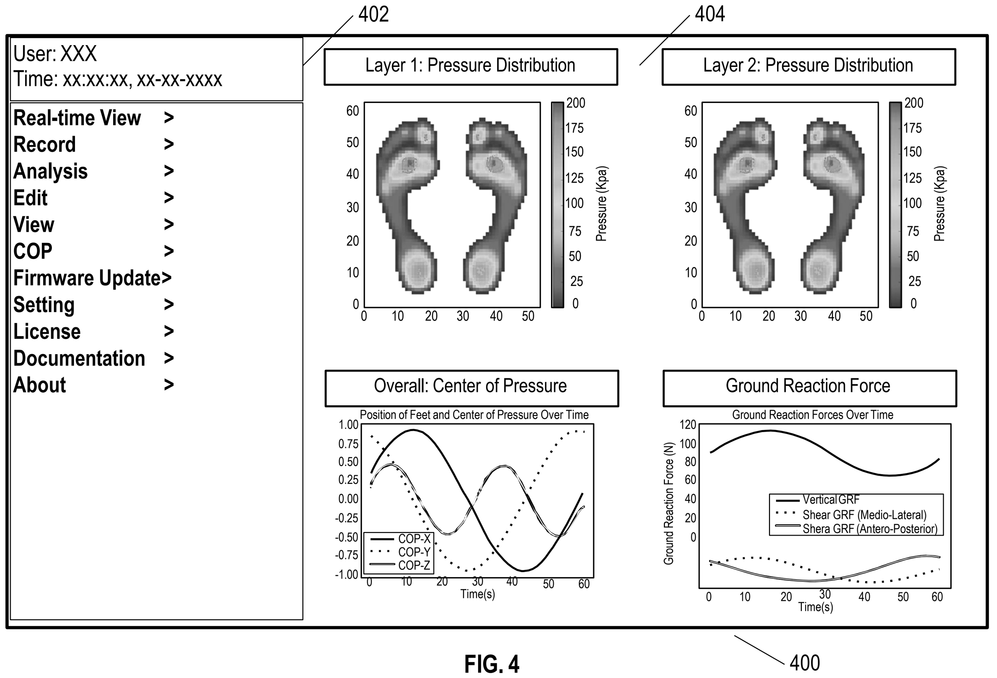

FIG. 4 illustrates an example display 400, in accordance with an example implementation. In some example implementations, the display 400 is a graphical user interface (GUI) accessed by a user on a user device (e.g., user device 220). As illustrated in FIG. 4, the display 400 may display information such as, but not limited to, a panel 402 and graphic display area 404. Selectable information as contained in the panel 402 include, but not limited to, real-time view, record, analysis, edit, view, Center of Pressure (CoP), etc. Other displayable information may include firmware information, setting, license, documentation, etc. The graphic display area 404 may include graphic information illustrating difference layers of pressure distribution of the insole layers, CoP position over time, GRF over time, etc.

The foregoing example implementation may have various benefits and advantages, such as measurement of the GRF between the feet and the ground through use of the multilayer insole structure. The unique combination and arrangement of sensors provides a way for measuring the share (horizontal) force component underneath the user's feet that is not available in any conventional technology. Further, the combination and arrangement of sensors requires minimal effort to set up, and may be utilized in existing shoe products. The measured GRF can be used to further monitor and analyze ergonomics in industrial operations, preventing potential injuries.
there is no technology available for measuring the shear (horizontal) force underneath the feet. Specifically, conventional technologies only focus on pressure distribution directly under the human foot. There is no clear quantitative measurement of the GRF between the feet and the ground.

Present example implementations relate to methods and systems for tracking and analyzing correlation between an individual/user's feet. For example, while a person is walking, it would be possible to track which foot is in front and determine its position in relation to the other foot. Example implementations generate information required for performing comprehensive biomechanical analysis of the whole body. In addition, example implementations also estimate a center of pressure for user's feet, which is a critical component in performing balance assessment. Coordination between an individual/user's feet is critical in assessing the whole human musculoskeletal system as it provides information such as, but not limited to, pose, load distribution, and the exact external forces applied to the feet, which are necessary to perform comprehensive biomechanical analysis of the full body.

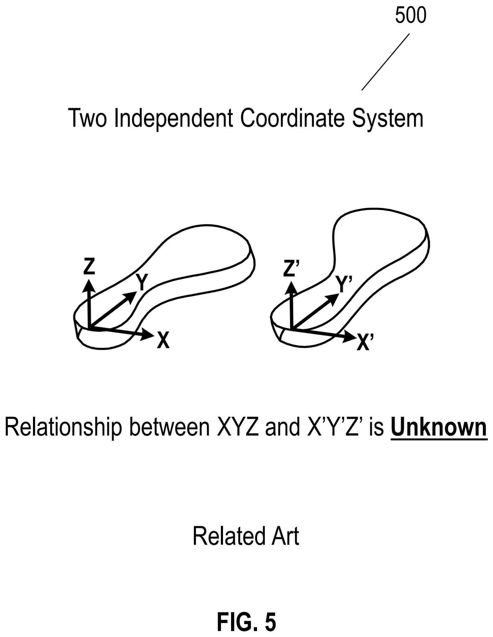

FIG. 5 illustrates two conventional smart sole systems 500. While it is possible for each of the smart sole systems 500 to generate and report data on a respective foot, the relationship between the coordinate system on both feet ((X, Y, Z) and (X', Y', Z')) cannot be discovered or tracked using the smart sole systems 500. For example, while pressure distribution in each foot can be measured and mapped individually, the geometrical relationship (e.g., distance, arrangement, etc.) between the high-pressure point(s) under the left foot and the high-pressure point(s) under the right foot cannot be determined. No identifiable correlation can be estimated when the two smart sole systems 500 are used.

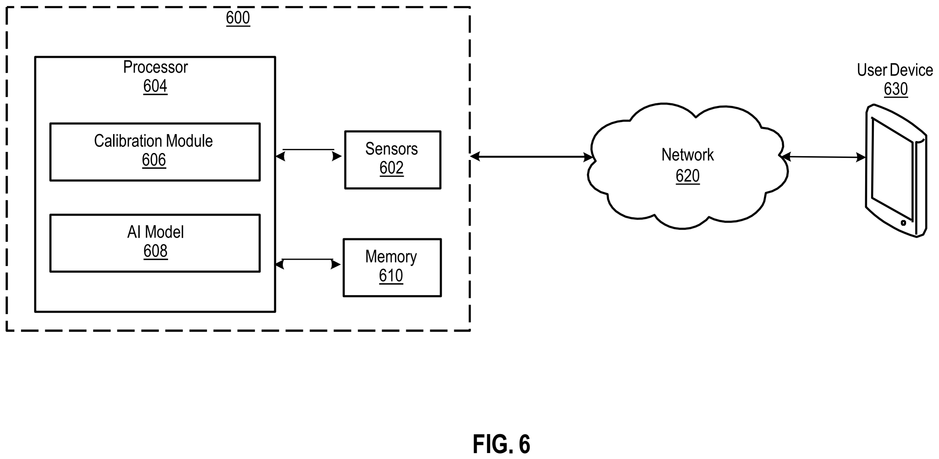

FIG. 6 illustrates an example system architecture 600 of a Unified Coordinate System (UCS) , in accordance with an example implementation. As illustrated in FIG. 6, the system architecture 600 may include components such as, but not limited to, sensors 602, a processor 604, etc. In some alternate implementations, different and/or additional components may be included in the system architecture 600.

The sensors 602 may be embedded in the insoles, midsoles, or outsoles of a pair of shoes. The sensors 602 may include either (i) at least one inertial measuring unit (IMU) sensor in each shoe; or (ii) a combination of IMU sensors and one of more of temperature sensors, humidity sensors, gyroscopes, accelerometers, magnetometers, etc., in the pair of shoes. The IMU sensors are used for measuring the acceleration, angular velocity, orientation, gravitational forces, etc., of the feet of the user while the user is performing one or more standardized activities. Sensors other than the IMU sensors may be incorporated to improve accuracy and/or provide additional information that are useful in performing further analysis. Three-dimensional reaction forces can be generated/measured through inclusion of additional sensors to the pressure sensors such that both vertical and shear reaction forces can be tracked. The measured sensor data is then transmitted from the sensors 602 to the processor 604.

In some example implementations, the processor 604 is a processor located on a server, which communicates with the sensors 602 through a network (e.g., network 620, etc.). Network 620 can be any network or combination of networks (e.g., internet, local area network, wide area network, telephonic network, cellular network, satellite network, etc.). The processor 604 may include components such as, but not limited to, a calibration module 606, an Artificial Intelligence (AI) module 608, etc.

The calibration module 606 performs calibration on the data received form the sensors 602 by comparing the data against a reference base. The calibration module 606 may also perform additional functions such as result testing and data validation to ensure that sensors 602 are working according to expectations and that desired results are being produced. Calibrated data is then generated by the calibration module 606 and received by the AI module 608. In some example implementations, the calibration module 606 also preprocesses the data by performing one or more of data cleaning, data organization, data transformation, data normalization, data reduction, etc. In alternate example implementations, the preprocessing is performed on the data by the processor 604 before the data is received by the calibration module 606.

The AI module 608 uses the calibrated data to generated a coordinate system with coordinates of key markers on both feet. First, the AI module 608 generates an initial position, a relative position, and an orientation of the feet of the user through the calibrated data. The AI module 608 then combines the initial position, the relative position, and the orientation of the feet of the user to generate a unified coordinate model. Coordinates of the key markers in the unified coordinate model are updated as sensor outputs are received during performance of the various standardized activities. Specifically, Coordinates of key markers in the left foot (X, Y, Z) and coordinates of key markers in the right food (X', Y', Z') are unified as coordinates (X", Y", Z"). Data/information is received in real-time by the calibration module 606 to track and update the positions and orientation of the feet in the unified coordinate model. Analyzation of the real-time data may be performed at a predetermined frequency (e.g., 1Hz, 1kHz, 1mHz, etc.) or based on user preference.

In some example implementations, the derivation and training of the unified coordinate model may be performed using historical data (e.g., sensor data, previously generated recommendations, etc.). The unified coordinate model may include, but not limited to, a convolutional neural network (CNN), a recurrent neural network (RNN), a deep RNN (DRNN), a Q-learning network (QN), a deep Q-learning network (DQN), decision trees, a K-Nearest Neighbors, etc. RNN may include long short-term memory (LSTM), etc.

The AI module 608 then utilizes the outputs of the unified coordinate model to further calculate a position of an overall Center of Pressure (CoP) of the user. In some example implementations, the AI module 608 further performs at least one of posture adjustment recommendation generation, evaluation report generation, or rehabilitation recommendation generation, etc., using the position of the overall CoP of the user. In some example implementations, the AI module 608 may further generate an AI recommendation model for generating the posture adjustment recommendation, evaluation report, or rehabilitation recommendation using the position of the overall CoP of the user.

In some example implementations, the calibrated data and the reports/recommendations generated by the AI module 608 are transmitted to a user device 630 for review. The information and recommendations may be presented in real-time through a graphic user interface (GUI) on the user device 630 as graphs, charts, texts, etc. In some example implementations, the sensor data, the calibrated data, the trained AI model, and the AI model outputs may be stored in a memory 610. The memory 610 may be any storage medium such as, but not limited to, read-only memories, random access memories, solid-state devices and drives, or any other types of tangible or non-transitory media suitable for storing electronic information. In alternate implementations, a remote storage may be used instead of the memory 610. For example, cloud storage may be utilized in performing the data storage function.

Examples of the user device 630 may include, but are not limited to, highly mobile devices (e.g., smartphones, devices in vehicles and other machines, devices carried by humans and animals, and the like), mobile devices (e.g., tablets, notebooks, laptops, personal computers, portable televisions, radios, and the like), and devices not designed for mobility (e.g., desktop computers, other computers, information kiosks, televisions with one or more processors embedded therein and/or coupled thereto, radios, and the like).

The user device 630 communicates with the system architecture 600/UCS through a network 620. Network 620 can be any network or combination of networks (e.g., internet, local area network, wide area network, telephonic network, cellular network, satellite network, etc.). In some example implementations, request for performing CoP computation/calculation may be issued by a user/operator through a user device 630 and received at the processor 604.

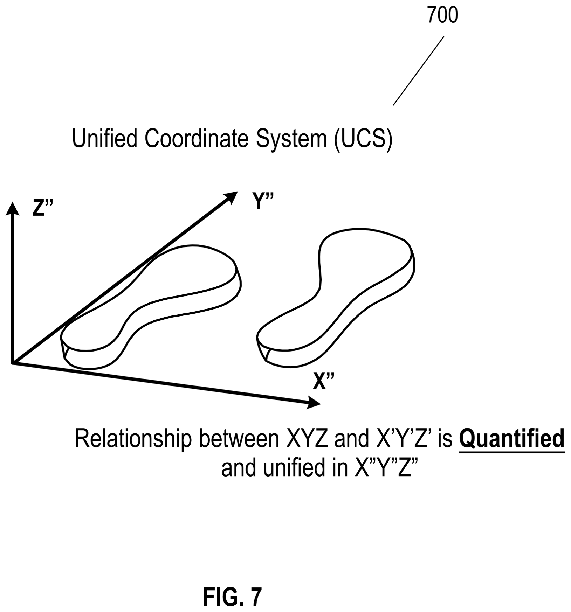

FIG. 7 illustrates an example smart sole system 700 utilizing the sensors 602, in accordance with an example implementation. Unlike the conventional smart sole systems 500, the smart sole system 700 is able to receive data from both fee so that coordinates of both feet may be unified and analyzed. Specifically, coordinates of the left foot (X, Y, Z) and coordinates of the right food (X', Y', Z') may be quantified and unified as coordinates (X", Y", Z"). The unified coordinates are identified as the CoP for both feet, which has various applications in the realm of biomechanical and ergonomics analysis. In addition, the CoP also contributes to more accurate calculation of the center of gravity.

The smart sole system 700 may form an integral part of a pair of shoes or be separate units/modules that inserted into common/conventional shoes (e.g., insoles, etc.). In some implementations, the smart sole system 700 may comprise a pair of sensor-embedded insoles for performing data collection.

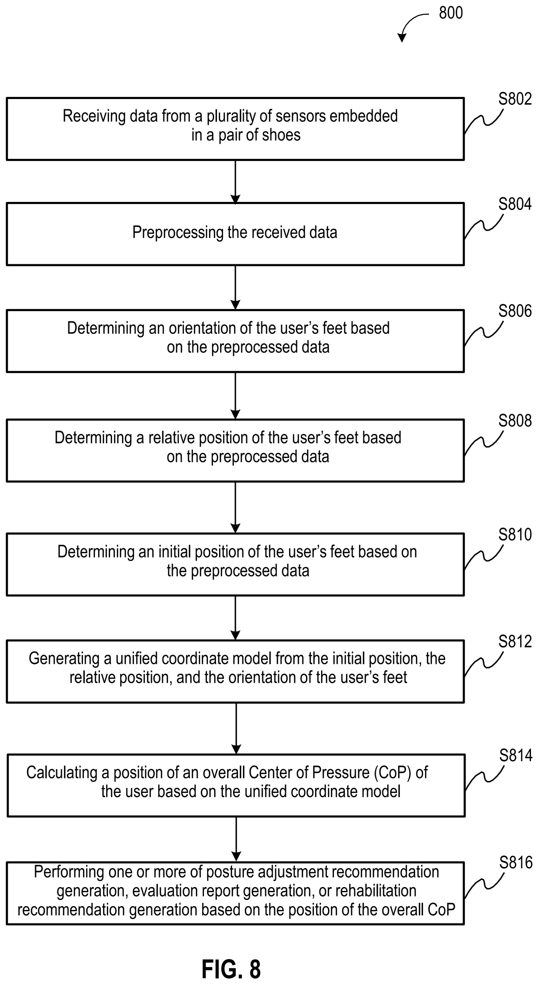

FIG. 8 illustrates an example process flow 800 for performing coordinate unification, in accordance with an example implementation. The process flow 800 begins at step S802 where data is received from a plurality of sensors (e.g., IMU sensors, etc.) embedded in a pair of shoes being used by a user in performing one or more standardized activities. In some example implementations, the plurality of sensors is embedded in insoles of the pair of shoes. The data may include data such as, but not limited to, data measuring linear acceleration along each axis, angular velocity/rotational rates around each axis, etc.

At step S804, the data is then preprocessed to generate preprocessed data. The data may be processed by performing one or more of data cleaning, data organization, data transformation, data normalization, data reduction, etc. At step S806, an orientation of the feet of the user is determined based on the data. At step S808, relative position of the feet of the user is determined based on the data. At step S810, an initial position of the feet of the user is determined based on the data. A unified coordinate model is then generated from the initial position, the relative position, and the orientation of the feet of the user at step S812. In some example implementations, one or more of (i) determination of the orientation of the feet of the user, (ii) determination of the relative position of the feet of the user, (iii) determination of the initial position of the feet of the user, or (iv) generation of the unified coordinate model may be performed using an AI model. The AI model may also be used to derive an initial position of the feet of the user through the data.

The AI model may include, but not limited to, recurrent neural network (RNN), deep RNN (DRNN), Q-learning network (QN), deep Q-learning network (DQN), decision trees, K-Nearest Neighbors, etc. RNN may include long short-term memory (LSTM), etc. In some example implementations, the AI model is a large multimodal language model that works with different types of input data, such as text, images, audio, video, etc. The AI model is iteratively trained using historical data as training input and training parameters are adjusted to determine the relative position of the feet, the orientation of the feet, and/or the unified coordinate model.

At step S814, a position of an overall Center of Pressure (CoP) of the user is then calculated based on the unified coordinate model. One or more of posture adjustment recommendation generation, evaluation report generation, or rehabilitation recommendation generation is then performed using the position of the overall CoP of the user at step S816. In some example implementations, the one or more reports or recommendations are generated using an AI model. The AI model is iteratively trained using historical data as training input and training parameters are adjusted to generate the posture adjustment recommendation, evaluation report, and the rehabilitation recommendation.

In some example implementations, the posture adjustment recommendation, evaluation report, and rehabilitation recommendation may be transmitted to a user device (e.g., user device 630) in real-time to be reviewed by the user. Examples of the user device may include, but are not limited to, mobile devices (e.g., smartphones, tablets, notebooks, laptops, personal computers, etc.) and devices not designed for mobility (e.g., desktop computers, information kiosks, televisions, etc.).

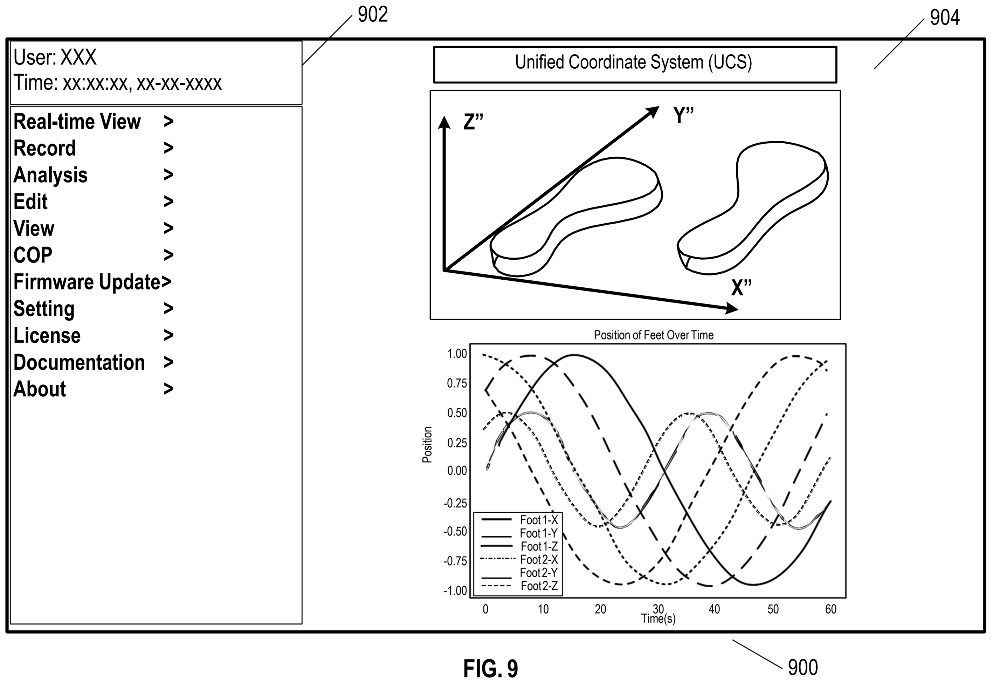

FIG. 9 illustrates an example display 900, in accordance with an example implementation. In some example implementations, the display 900 is a graphical user interface (GUI) accessed by a user on a user device (e.g., user device 630). As illustrated in FIG. 9, the display 900 may display information such as, but not limited to, a panel 902 and graphic display area 904. Selectable information as contained in the panel 902 include, but not limited to, real-time view, record, analysis, edit, view, Center of Pressure (CoP), etc. Other displayable information may include firmware information, setting, license, documentation, etc. The graphic display area 904 may include graphic information illustrating the Unified Coordinate System (UCS) in graphic form, CoP position over time, position of feet over time, etc.

The foregoing example implementation may have various benefits and advantages, such as improved CoP calculation/derivation through a unique combination and arrangement of sensors. Specifically, the CoP for both feet, a critical measurement in balance assessment and human musculoskeletal system assessment, can be more accurately calculated/derived from relative position and orientation of the feet through use of the UCS. While conventional smart insoles are capable of measuring the plantar pressure or vertical force component underneath user's feet, the measured data is generated on a per foot basis and incapable of being used to derive the CoP for both feet. The improved CoP in the lower body helps enhence comprehensive biomechanical analysis of the user's body (e.g., industrial ergo analysis and assessment, patient evaluation, balance analysis, musculoskeletal analysis, etc.)

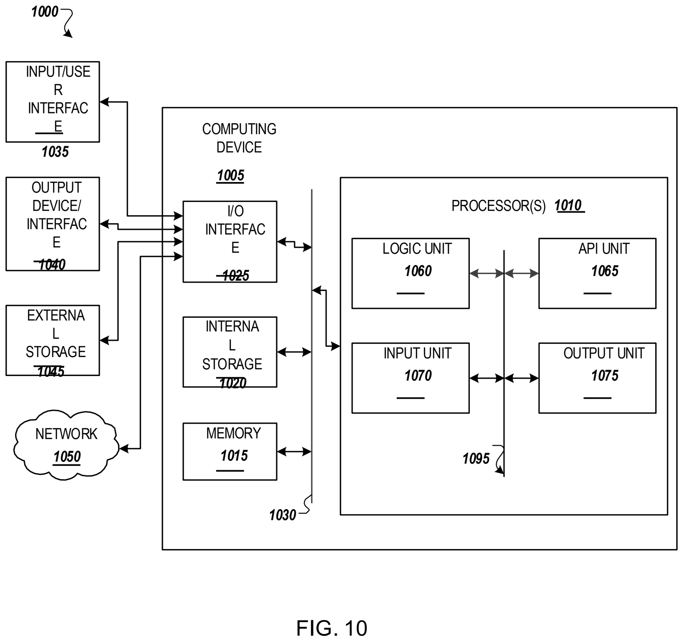

FIG. 10 illustrates an example computing environment with an example computer device suitable for use in some example implementations. Computer device 1005 in computing environment 1000 can include one or more processing units, cores, or processors 1010, memory 1015 (e.g., RAM, ROM, and/or the like), internal storage 1020 (e.g., magnetic, optical, solid-state storage, and/or organic), and/or IO interface 1025, any of which can be coupled on a communication mechanism or bus 1030 for communicating information or embedded in the computer device 1005. IO interface 1025 is also configured to receive images from cameras or provide images to projectors or displays, depending on the desired implementation.

Computer device 1005 can be communicatively coupled to input/user interface 1035 and output device/interface 1040. Either one or both of the input/user interface 1035 and output device/interface 1040 can be a wired or wireless interface and can be detachable. Input/user interface 1035 may include any device, component, sensor, or interface, physical or virtual, that can be used to provide input (e.g., buttons, touch-screen interface, keyboard, a pointing/cursor control, microphone, camera, braille, motion sensor, accelerometer, optical reader, and/or the like). Output device/interface 1040 may include a display, television, monitor, printer, speaker, braille, or the like. In some example implementations, input/user interface 1035 and output device/interface 1040 can be embedded with or physically coupled to the computer device 1005. In other example implementations, other computer devices may function as or provide the functions of input/user interface 1035 and output device/interface 1040 for a computer device 1005.

Examples of computer device 1005 may include, but are not limited to, highly mobile devices (e.g., smartphones, devices in vehicles and other machines, devices carried by humans and animals, and the like), mobile devices (e.g., tablets, notebooks, laptops, personal computers, portable televisions, radios, and the like), and devices not designed for mobility (e.g., desktop computers, other computers, information kiosks, televisions with one or more processors embedded therein and/or coupled thereto, radios, and the like).

Computer device 1005 can be communicatively coupled (e.g., via IO interface 1025) to external storage 1045 and network 1050 for communicating with any number of networked components, devices, and systems, including one or more computer devices of the same or different configuration. Computer device 1005 or any connected computer device can be functioning as, providing services of, or referred to as a server, client, thin server, general machine, special-purpose machine, or another label.

IO interface 1025 can include but is not limited to, wired and/or wireless interfaces using any communication or IO protocols or standards (e.g., Ethernet, 802.11x, Universal System Bus, WiMax, modem, a cellular network protocol, and the like) for communicating information to and/or from at least all the connected components, devices, and network in computing environment 1000. Network 1050 can be any network or combination of networks (e.g., the Internet, local area network, wide area network, a telephonic network, a cellular network, satellite network, and the like).

Computer device 1005 can use and/or communicate using computer-usable or computer readable media, including transitory media and non-transitory media. Transitory media include transmission media (e.g., metal cables, fiber optics), signals, carrier waves, and the like. Non-transitory media include magnetic media (e.g., disks and tapes), optical media (e.g., CD ROM, digital video disks, Blu-ray disks), solid-state media (e.g., RAM, ROM, flash memory, solid-state storage), and other non-volatile storage or memory.

Computer device 1005 can be used to implement techniques, methods, applications, processes, or computer-executable instructions in some example computing environments. Computer-executable instructions can be retrieved from transitory media, and stored on and retrieved from non-transitory media. The executable instructions can originate from one or more of any programming, scripting, and machine languages (e.g., C, C++, C#, Java, Visual Basic, Python, Perl, JavaScript, and others).

Processor(s) 1010 can execute under any operating system (OS) (not shown), in a native or virtual environment. One or more applications can be deployed that include logic unit 1060, application programming interface (API) unit 1065, input unit 1070, output unit 1075, and inter-unit communication mechanism 1095 for the different units to communicate with each other, with the OS, and with other applications (not shown). The described units and elements can be varied in design, function, configuration, or implementation and are not limited to the descriptions provided. Processor(s) 1010 can be in the form of hardware processors such as central processing units (CPUs) or in a combination of hardware and software units.

In some example implementations, when information or an execution instruction is received by API unit 1065, it may be communicated to one or more other units (e.g., logic unit 1060, input unit 1070, output unit 1075). In some instances, logic unit 1060 may be configured to control the information flow among the units and direct the services provided by API unit 1065, the input unit 1070, the output unit 1075, in some example implementations described above. For example, the flow of one or more processes or implementations may be controlled by logic unit 1060 alone or in conjunction with API unit 1065. The input unit 1070 may be configured to obtain input for the calculations described in the example implementations, and the output unit 1075 may be configured to provide an output based on the calculations described in example implementations.

Processor(s) 1010 can be configured to receive raw data from a plurality of sensors embedded in a pair of shoes being used by a user as shown in FIGS. 1 to 4. The processor(s) 1010 may also be configured to perform preprocessing of the raw data to generate preprocessed data as shown in FIGS. 1 to 4. The processor(s) 1010 may also be configured to calculate ground reaction force on the user using the preprocessed data as shown in FIGS. 1 to 4. The processor(s) 1010 may also be configured to perform at least one of posture adjustment recommendation generation, report generation, or additional data analysis using the ground reaction force derived from the preprocessed data as shown in FIGS. 1 to 4.

The processor(s) 1010 may also be configured to receive data from a plurality of sensors embedded in a pair of shoes while the pair of shoes are being used in one or more standardized activities by a user as shown in FIGS. 6 to 9. The processor(s) 1010 may also be configured to determine relative position of feet of the user based on the data as shown in FIGS. 6 to 9. The processor(s) 1010 may also be configured to generate a unified coordinate model from the relative position of the feet of the user as shown in FIGS. 6 to 9. The processor(s) 1010 may also be configured to calculate a position of an overall Center of Pressure (CoP) of the user based on the unified coordinate model as shown in FIGS. 6 to 9.

The processor(s) 1010 may also be configured to determine orientation of the feet of the user based on the data as shown in FIGS. 6 to 9. The processor(s) 1010 may also be configured to perform at least one of posture adjustment recommendation generation, evaluation report generation, or rehabilitation recommendation generation using the position of the overall CoP of the user as shown in FIGS. 6 to 9.

Some portions of the detailed description are presented in terms of algorithms and symbolic representations of operations within a computer. These algorithmic descriptions and symbolic representations are the means used by those skilled in the data processing arts to convey the essence of their innovations to others skilled in the art. An algorithm is a series of defined steps leading to a desired end state or result. In example implementations, the steps carried out require physical manipulations of tangible quantities for achieving a tangible result.

Unless specifically stated otherwise, as apparent from the discussion, it is appreciated that throughout the description, discussions utilizing terms such as "processing," "computing," "calculating," "determining," "displaying," or the like, can include the actions and processes of a computer system or other information processing device that manipulates and transforms data represented as physical (electronic) quantities within the computer system's registers and memories into other data similarly represented as physical quantities within the computer system's memories or registers or other information storage, transmission or display devices.

Example implementations may also relate to an apparatus for performing the operations herein. This apparatus may be specially constructed for the required purposes, or it may include one or more general-purpose computers selectively activated or reconfigured by one or more computer programs. Such computer programs may be stored in a computer readable medium, such as a computer readable storage medium or a computer readable signal medium. A computer readable storage medium may involve tangible mediums such as, but not limited to optical disks, magnetic disks, read-only memories, random access memories, solid-state devices, and drives, or any other types of tangible or non-transitory media suitable for storing electronic information. A computer readable signal medium may include mediums such as carrier waves. The algorithms and displays presented herein are not inherently related to any particular computer or other apparatus. Computer programs can involve pure software implementations that involve instructions that perform the operations of the desired implementation.

Various general-purpose systems may be used with programs and modules in accordance with the examples herein, or it may prove convenient to construct a more specialized apparatus to perform desired method steps. In addition, the example implementations are not described with reference to any particular programming language. It will be appreciated that a variety of programming languages may be used to implement the teachings of the example implementations as described herein. The instructions of the programming language(s) may be executed by one or more processing devices, e.g., central processing units (CPUs), processors, or controllers.

As is known in the art, the operations described above can be performed by hardware, software, or some combination of software and hardware. Various aspects of the example implementations may be implemented using circuits and logic devices (hardware), while other aspects may be implemented using instructions stored on a machine-readable medium (software), which if executed by a processor, would cause the processor to perform a method to carry out implementations of the present application. Further, some example implementations of the present application may be performed solely in hardware, whereas other example implementations may be performed solely in software. Moreover, the various functions described can be performed in a single unit, or can be spread across a number of components in any number of ways. When performed by software, the methods may be executed by a processor, such as a general-purpose computer, based on instructions stored on a computer readable medium. If desired, the instructions can be stored on the medium in a compressed and/or encrypted format.

Moreover, other implementations of the present application will be apparent to those skilled in the art from consideration of the specification and practice of the teachings of the present application. Various aspects and/or components of the described example implementations may be used singly or in any combination. It is intended that the specification and example implementations be considered as examples only, with the true scope and spirit of the present application being indicated by the following claims.

## Claims

1. A pressure profiling method, the method comprising:
receiving, by a processor, raw data from a plurality of sensors embedded in a pair of shoes being used by a user;
performing, by the processor, preprocessing of the raw data to generate preprocessed data;
calculating, by the processor, ground reaction force on the user using the preprocessed data; and
performing, by the processor, at least one of posture adjustment recommendation generation, report generation, or additional data analysis using the ground reaction force derived from the preprocessed data.

2. The method of claim 1, wherein the plurality of sensors are embedded in insoles and at least one of midsoles or outsoles of the pair of shoes,
wherein the plurality of sensors captures a pressure profile between feet of the user and an external contacting surface of the pair of shoes as the raw data.

3. The method of claim 2, wherein the midsoles comprise at least one midsole layer in each shoe of the pair of shoes.

4. The method of claim 1, wherein the raw data comprises either (i) data measuring vertical reaction force on feet of the user; or (ii) data measuring both the vertical reaction force and shear reaction force on the feet of the user.

5. The method of claim 1, wherein the plurality of sensors comprises either (i) pressure sensors; or (ii) the pressure sensors and one or more of inertial measuring unit (IMU) sensors, temperature sensors, or humidity sensors.

6. The method of claim 1, wherein the processor is configured to perform the at least one of posture adjustment recommendation generation, report generation, or additional data analysis using an Artificial Intelligence (AI) model.

7. A pressure profiling system, the system comprising:
a plurality of sensors; and
a processor in communication with the plurality of sensors, wherein the processor is configured to:
receive raw data from the plurality of sensors, the plurality of sensors is embedded in a pair of shoes being used by a user;
perform preprocessing of the raw data to generate preprocessed data;
calculate ground reaction force on the user using the preprocessed data; and
perform at least one of posture adjustment recommendation generation, report generation, or additional data analysis using the ground reaction force derived from the preprocessed data.

8. The system of claim 7, wherein the plurality of sensors are embedded in insoles and at least one of midsoles or outsoles of the pair of shoes,
wherein the plurality of sensors captures a pressure profile between feet of the user and an external contacting surface of the pair of shoes as the raw data.

9. The system of claim 8, wherein the midsoles comprise at least one midsole layer in each shoe of the pair of shoes.

10. The system of claim 7, wherein the raw data comprises either (i) data measuring vertical reaction force on feet of the user; or (ii) data measuring both the vertical reaction force and shear reaction force on the feet of the user.

11. The system of claim 7, wherein the processor is configured to perform the at least one of posture adjustment recommendation generation, report generation, or additional data analysis using an Artificial Intelligence (AI) model.

12. A coordinate unification method, the method comprising:
receiving, by a processor, data from a plurality of sensors embedded in a pair of shoes while the pair of shoes are being used in one or more standardized activities by a user;
determining, by the processor, relative position of feet of the user based on the data;
generating, by the processor, a unified coordinate model from the relative position of the feet of the user; and
calculating, by the processor, a position of an overall Center of Pressure (CoP) of the user based on the unified coordinate model.

13. The method of claim 12, further comprising:
determining, by the processor, orientation of the feet of the user based on the data,
wherein the processor is configured to generate the unified coordinate model from both the relative position of the feet of the user and the orientation of the feet.

14. The method of claim 13, wherein the processor is configured to determine the relative position and the orientation of the feet of the user by using an Artificial Intelligence (AI) model,
wherein the AI model derives an initial position, the relative position, and the orientation of the feet of the user through the data.

15. The method of claim 12, further comprising:
performing, by the processor, at least one of posture adjustment recommendation generation, evaluation report generation, or rehabilitation recommendation generation using the position of the overall CoP of the user.

16. The method of claim 12, wherein the plurality of sensors are embedded in insoles of the pair of shoes.

17. The method of claim 12, wherein the plurality of sensors comprises a plurality of inertial measuring unit (IMU) sensors.

18. A coordinate unification system, the system comprising:
a plurality of sensors; and
a processor in communication with the plurality of sensors, wherein the processor is configured to:
receive data from the plurality of sensors embedded in a pair of shoes while the pair of shoes are being used in one or more standardized activities by a user;
determine relative position of feet of the user based on the data;
generate a unified coordinate model from the relative position of the feet of the user; and
calculate a position of an overall Center of Pressure (CoP) of the user based on the unified coordinate model.

19. The system of claim 18, wherein the processor is further configured to:
determine orientation of the feet of the user based on the data,
wherein the processor is configured to generate the unified coordinate model from both the relative position of the feet of the user and the orientation of the feet,
wherein the processor is configured to determine the relative position and the orientation of the feet of the user by using an Artificial Intelligence (AI) model,
wherein the AI model derives an initial position, the relative position, and the orientation of the feet of the user through the data.

20. The system of claim 18, wherein the processor is further configured to:
perform at least one of posture adjustment recommendation generation, evaluation report generation, or rehabilitation recommendation generation using the position of the overall CoP of the user.
